# EUROPÄISCHE PATENTANMELDUNG

(11) **EP 2 305 665 A1**
(43) Veröffentlichungstag der Anmeldung: **06.04.2011**
(21) Anmeldenummer: 10180840.0
(22) Anmeldetag: 01.06.2006
(51) Int. Cl.: C07D 401/12, A61K 31/4402, A61K 31/4184

(54) **Polymorphe von 3-[(2-{[4-(Hexyloxycarbonylamino- imino-methyl)-phenylamino]-methyl}-1-methyl-1H-benzimidazol-5-carbonyl)-pyridin-2-yl-amino]-propionsäure-ethylester**

(30) Priorität: 04.06.2005 DE 102005025728
(62) Teilanmeldung aus: 09157302.2
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Sieger, Peter, 55216 INGELHEIM AM RHEIN (DE); Hauel, Norbert, 55216 INGELHEIM AM RHEIN (DE); Schmid, Rolf, 55216 INGELHEIM AM RHEIN (DE); Sobotta, Rainer, 55216 INGELHEIM AM RHEIN (DE)
(74) Vertreter: Hammann, Heinz

(57) **Zusammenfassung**

Die Erfindung betrifft neue Polymorphe des Wirkstoffs 3-[(2-{[4-(Hexyloxycarbonyl-amino-imino-methyl)-phenylamino]-methyl}-1-methyl-1*H*-benzimidazol-5-carbonyl)-pyridin-2-yl-amino]-propionsäure-ethylester, deren Herstellung und deren Verwendung als Arzneimittel.

## Beschreibung

### Polymorphe von 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester

Die Erfindung betrifft neue Polymorphe des Wirkstoffs 3-[(2-{[4-(Hexyloxycarbonyl-amino-imino-methyl)-phenylamino]-methyl}-1-methyl-1*H*-benzimidazol-5-carbonyl)-pyridin-2-yl-amino]-propionsäure-ethylester, Verfahren zu ihrer Herstellung und deren Verwendung als Arzneimittel. Dieser Wirkstoff mit der chemischen Formel 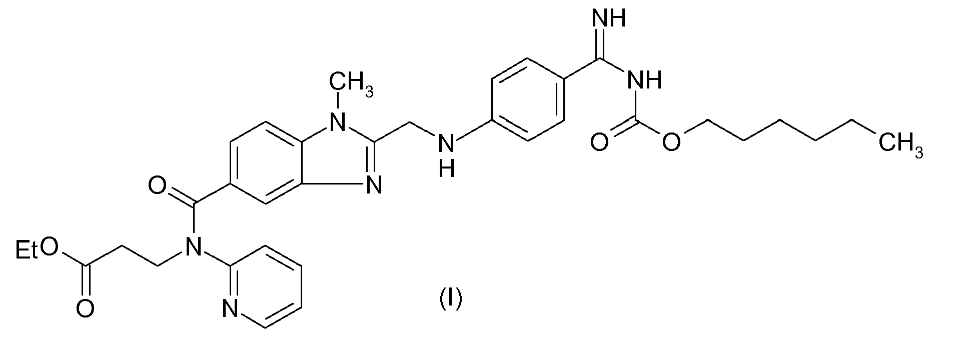 ist bereits aus der WO 98/37075, in der Verbindungen mit einer Thrombin-hemmenden und die Thrombinzeit verlängernden Wirkung offenbart werden, unter dem Namen 1-Methyl-2-[*N*-[4-(*N*-n-hexyloxycarbonylamidino)phenyl]-amino-methyl]-benzimidazol-5-yl-carbonsäure-*N*-(2-pyridyl)-*N*-(2-ethoxycarbonylethyl)-amid bekannt. Bei der Verbindung der Formel I handelt es sich um ein Doppel-Prodrug der Verbindung 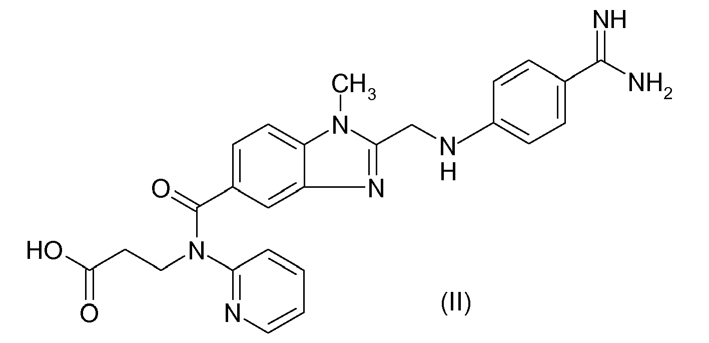 d.h. die Verbindung der Formel I wird erst im Körper in die eigentlich wirksame Verbindung, nämlich die Verbindung der Formel II, umgewandelt. Hauptindikationsgebiet der Verbindung der chemischen Formel I ist die postoperative Prophylaxe von tiefen Venenthrombosen und die Prophylaxe von Schlaganfällen.

Aufgabe der Erfindung ist es, neue Polymorphe der Verbindung der Formel I mit vorteilhaften Eigenschaften für die pharmazeutische Anwendung bereitzustellen.

Die vorstehend genannten pharmakologisch wertvollen Eigenschaften der im Stand der Technik offenbarten disubstituierten bicyclischen Heterocyclen stellen die Grundvoraussetzung für eine wirksame Verwendung der Verbindungen als Arzneimittel dar. Neben der eigentlichen Wirksamkeit für die gewünschte Indikation muß ein Wirkstoff noch weiteren Anforderungen gerecht werden, um als Arzneimittel zum Einsatz gelangen zu können. Diese Parameter sind zu einem großen Teil mit der physikochemischen Beschaffenheit des Wirkstoffs verbunden.

Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der Arzneimittelzusammensetzungen verwendete Arzneiwirkstoff sollte daher eine hohe Stabilität aufweisen, die auch unter verschiedenen Umgebungsbedingungen gewährleistet sein muß. Dies ist zwingend erforderlich, um zu verhindern, dass Arzneimittelzusammensetzungen Verwendung finden, in denen neben tatsächlichem Wirkstoff beispielsweise Abbauprodukte desselben enthalten sind. In einem solchen Fall könnte ein in pharmazeutischen Formulierungen vorgefundener Gehalt an Wirkstoff niedriger sein als spezifiziert.

Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird. Vorzugsweise sollte ein Arzneimittelwirkstoff daher nur in geringem Maße hygroskopisch sein.

Da die Kristallmodifikation eines Wirkstoffs für den reproduzierbaren Wirkstoffgehalt einer Darreichungsform von Bedeutung ist, besteht die Notwendigkeit, eventuell existierenden Polymorphismus eines kristallin vorliegenden Wirkstoffs bestmöglich aufzuklären. Sofern verschiedene polymorphe Modifikationen eines Wirkstoffs auftreten, sollte gewährleistet sein, dass sich die kristalline Modifikation der Substanz in der späteren Arzneimittelzubereitung nicht verändert. Andernfalls könnte dies die reproduzierbare Wirksamkeit des Medikaments nachteilig beeinflussen.

Ein weiteres Kriterium, welches je nach Wahl der Formulierung oder nach Wahl des Herstellungsverfahrens der Formulierung von unter Umständen herausragender Bedeutung ist, ist die Löslichkeit des Wirkstoffs. Werden beispielsweise Arzneimittellösungen (etwa für Infusionen) bereitgestellt, so ist eine ausreichende Löslichkeit des Wirkstoffs in physiologisch verträglichen Lösemitteln unverzichtbar. Auch für oral zu applizierende Arzneimittel ist eine ausreichende Löslichkeit des Wirkstoffs von großer Wichtigkeit.

Der vorliegenden Erfindung liegt die Aufgabe zugrunde, einen Arzneimittelwirkstoff bereitzustellen, der nicht nur durch eine hohe pharmakologische Wirksamkeit gekennzeichnet ist, sondern ferner den vorstehend genannten physikochemischen Anforderungen bestmöglich gerecht wird.

Überraschenderweise wurde nun gefunden, dass die erfindungsgemäßen Polymorphe der Verbindung der Formel I (Dabigatran etexilate) diese Aufgabe erfüllen und vorteilhafte Eigenschaften aufweisen.

Gegenstand der Erfindung sind daher die Polymorphe von 3-[(2-{[4-(Hexyloxy-carbonylamino-imino-methyl)-phenylamino]-methyl}-1-methyl-1*H*-benzimidazol-5-carbonyl)-pyridin-2-yl-amino]-propionsäure-ethylester mit den Bezeichnungen wasserfreie Form I, wasserfreie Form II und Tetrahydrat. Ein weiterer Erfindungsgegenstand sind pharmazeutische Zusammensetzungen enthaltend mindestens eines der oben genannten Polymorphe sowie Verfahren zur Herstellung von Arzneimitteln, welche zur Prophylaxe von Venenthrombosen und Schlaganfall geeignet sind und die erfindungsgemäßen Polymorphe enthalten.

Ein erster Gegenstand der vorliegenden Erfindung sind daher die drei oben erwähnten polymorphen Formen des Wirkstoffs 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester, bevorzugt in kristalliner Form, gekennzeichnet durch Schmelzpunkte von T_{Smp}. = 135 ± 3°C (wasserfreie Form I), T_{Smp}. = 150 ± 3°C (wasserfreie Form II) bzw. T_{Smp}. = 90 ± 5°C (Tetrahydrat) (bestimmt über DSC; Auswertung über Peak-Maximum; Heizrate: 10°C/min). Das DSC-Diagramm der wasserfreien Form I zeichnet sich dadurch aus, dass vier weitere schwach endotherme Signale bei ca. 53, 75, 98 und 118°C zu beobachten sind. Diese Signale sind auf voll reversible fest-fest-Phasenübergänge zurückzuführen, d.h. im Temperaturbereich zwischen 53 - 75, 75 - 98, 98 - 118 und 118 - 135 °C existieren vier weitere Hochtemperaturphasen der wasserfreien Form I.

Ein weiterer Erfindungsgegenstand sind die Verfahren zur selektiven Herstellung der drei polymorphen Formen sowie die nach diesen Verfahren erhältlichen Modifikationen.

Erfindungsgemäß erhält man die wasserfreie Form I von 3-[(2-{[4-(Hexyloxycarbonyl-amino-imino-methyl)-phenylamino]-methyl}-1-methyl-1*H*-benzimidazol-5-carbonyl)-pyridin-2-yl-amino]-propionsäure-ethylester, indem man
a) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base unter Rückfluß in Essigsäureethylester löst,
b) die Lösung auf eine Temperatur von ca. 30°C bis 35 °C abkühlt und weitere 60 Minuten bei dieser Temperatur rührt,
c) auf ca. 15°C bis 20 °C abkühlt und weitere etwa 60 Minuten lang bei dieser Temperatur nachrührt,
d) die ausgefallenen Kristalle absaugt, mit Essigsäureethylester wäscht und
e) das so erhaltene Produkt bei 40 bis 50 °C im Umluft-Trockenschrank trocknet.

Erfindungsgemäß erhält man die wasserfreie Form II von 3-[(2-{[4-(Hexyloxy-carbonylamino-imino-methyl)-phenylamino]-methyl}-1-methyl-1*H*-benzimidazol-5-carbonyl)-pyridin-2-yl-amino]-propionsäure-ethylester, indem man
a) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base mit wenig Essigsäureethylester versetzt,
b) die so erhaltene Suspension auf eine Temperatur von ca. 80 °C erwärmt, wobei eine klare Lösung entsteht,
c) die Lösung ca. eine Stunde lang unter Rückfluß erhitzt,
d) die ausgefallenen Kristalle abfiltriert und
e) das so erhaltene Produkt an der Luft trocknet.

Erfindungsgemäß erhält man das Tetrahydrat von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester, indem man
a) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base unter Schütteln bei einer Temperatur von ca. 60 °C in Aceton/Wasser (80 : 20) löst,
b) die Lösung auf eine Temperatur von ca. 30 °C abkühlt und in ein verschließbares Gefäß filtriert,
c) das verschlossene Gefäß mit der Lösung auf eine Temperatur von ca. - 9 °C abkühlt und ca. 30 Minuten lang bei dieser Temperatur beläßt,
d) ein auf -9 °C vorgekühltes Gemisch aus Aceton und Wasser (80 : 20) zugibt und die Mischung aufschüttelt,
e) die ausgefallenen Kristalle absaugt und mit einem auf -9 °C gekühlten Gemisch aus Aceton und Wasser (80 : 20) wäscht und
f) das so erhaltene Produkt an der Luft bei Raumtemperatur trocknet.

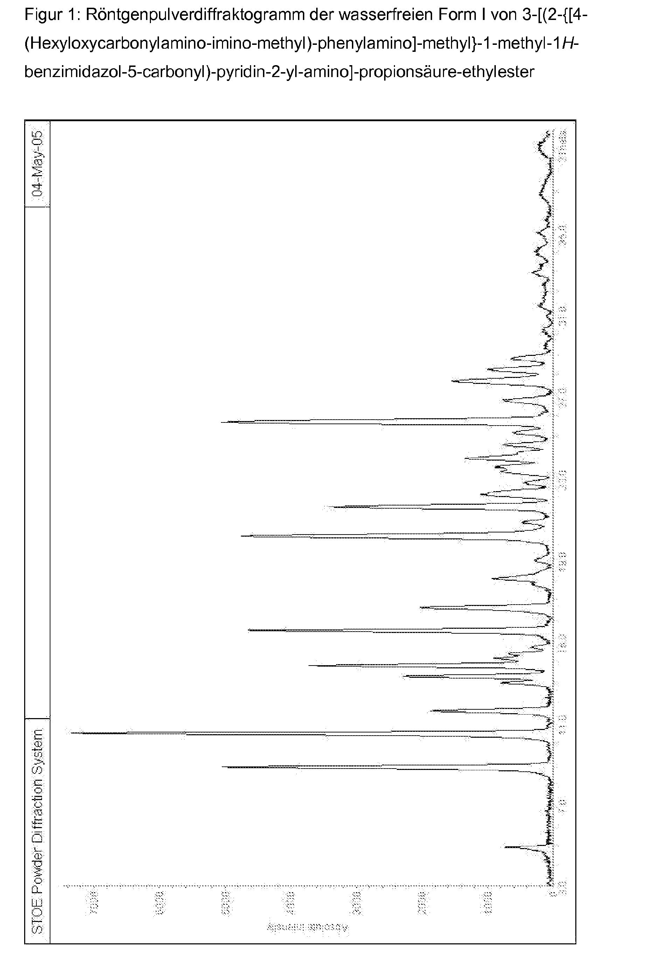

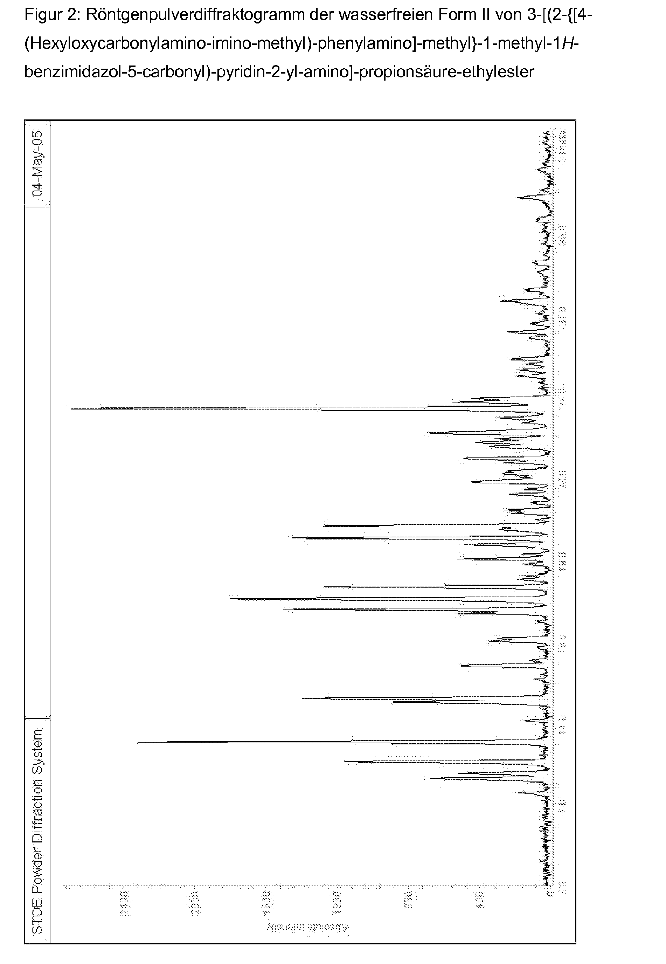

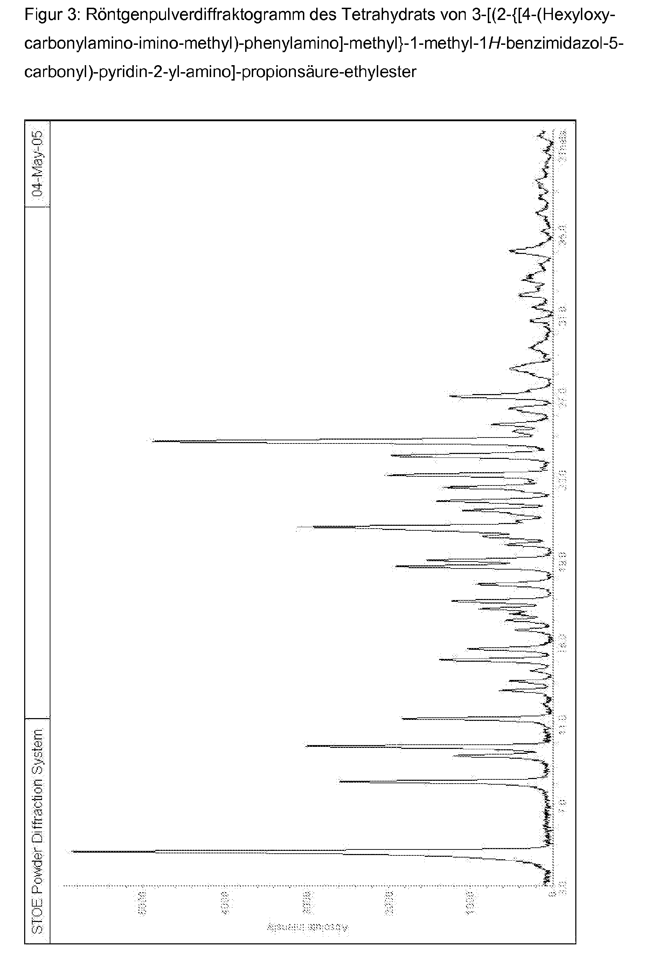

Die erfindungsgemäßen kristallinen Formen von 3-[(2-{[4-(Hexyloxycarbonylamino-imino-methyl)-phenylamino]-methyl}-1-methyl}-1*H*-benzimidazol-5-carbonyl)-pyridin-2-yl-amino]-propionsäure-ethylester wurden mittels Röntgenpulverbeugung näher untersucht. Die erhaltenen Diagramme sind in Figuren 1 bis 3 dargestellt.

Die nachstehenden Tabellen 1 bis 3 führen die bei dieser Analyse erhaltenen Daten auf:

**Tabelle 1: Röntgenpulverreflexe (bis zu 30 ° 2Θ) und Intensitäten (normalisiert) von 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester (wasserfreie Form I)**

| **2 Θ [°]** | **d [Å]** | **I/Iₒ [%]** |
|---|---|---|
| 3,39 | 26,07 | 1 |
| 4,31 | 20,51 | 1 |
| 4,87 | 18,12 | 10 |
| 5,62 | 15,70 | 1 |
| 7,28 | 12,14 | 1 |
| 7,43 | 11,88 | 1 |
| 8,82 | 10,02 | 69 |
| 10,46 | 8,45 | 100 |
| 11,56 | 7,65 | 25 |
| 12,92 | 6,84 | 11 |
| 13,25 | 6,67 | 32 |
| 13,78 | 6,42 | 50 |
| 14,12 | 6,27 | 12 |
| 14,36 | 6,16 | 9 |
| 14,67 | 6,03 | 5 |
| 15,49 | 5,72 | 63 |
| 16,61 | 5,33 | 28 |
| 17,76 | 4,99 | 4 |
| 18,03 | 4,92 | 12 |
| 18,93 | 4,69 | 4 |
| 20,12 | 4,41 | 65 |
| 20,79 | 4,27 | 6 |
| 21,54 | 4,12 | 46 |
| 22,15 | 4,01 | 15 |
| 22,72 | 3,91 | 6 |
| 23,28 | 3,82 | 11 |
| 23,51 | 3,78 | 11 |
| 23,92 | 3,72 | 18 |
| 24,18 | 3,68 | 8 |
| 24,58 | 3,62 | 10 |
| 25,18 | 3,53 | 8 |
| 25,70 | 3,46 | 69 |
| 26,75 | 3,33 | 10 |
| 27,69 | 3,22 | 21 |
| 28,27 | 3,15 | 14 |
| 28,80 | 3,10 | 9 |
| 29,41 | 3,03 | 2 |
| 30,16 | 2,96 | 2 |

**Tabelle 2: Röntgenpulverreflexe (bis zu 30 ° 2Θ) und Intensitäten (normalisiert) von 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester (wasserfreie Form II)**

| **2 Θ [°]** | **d [Å]** | **I/Iₒ [%]** |
|---|---|---|
| 7,55 | 11,70 | 6 |
| 8,25 | 10,71 | 24 |
| 8,50 | 10,40 | 18 |
| 9,06 | 9,75 | 44 |
| 10,04 | 8,80 | 86 |
| 11,08 | 7,98 | 5 |
| 11,99 | 7,37 | 33 |
| 12,17 | 7,27 | 51 |
| 13,11 | 6,75 | 4 |
| 13,77 | 6,42 | 19 |
| 14,04 | 6,30 | 5 |
| 14,67 | 6,04 | 3 |
| 14,98 | 5,91 | 13 |
| 15,10 | 5,86 | 11 |
| 15,93 | 5,56 | 3 |
| 16,34 | 5,42 | 20 |
| 16,52 | 5,36 | 55 |
| 17,03 | 5,20 | 68 |
| 17,63 | 5,03 | 47 |
| 17,99 | 4,93 | 7 |
| 18,17 | 4,88 | 5 |
| 18,74 | 4,73 | 6 |
| 19,00 | 4,67 | 19 |
| 19,23 | 4,61 | 6 |
| 19,69 | 4,51 | 17 |
| 20,02 | 4,43 | 53 |
| 20,43 | 4,34 | 11 |
| 20,63 | 4,30 | 48 |
| 20,92 | 4,24 | 3 |
| 21,24 | 4,18 | 7 |
| 21,39 | 4,15 | 10 |
| 21,75 | 4,08 | 4 |
| 22,15 | 4,01 | 9 |
| 22,41 | 3,96 | 6 |
| 22,77 | 3,90 | 17 |
| 23,02 | 3,86 | 6 |
| 23,17 | 3,84 | 10 |
| 23,70 | 3,75 | 10 |
| 23,92 | 3,72 | 19 |
| 24,49 | 3,63 | 13 |
| 24,69 | 3,60 | 15 |
| 24,89 | 3,57 | 12 |
| 25,17 | 3,53 | 26 |
| 25,66 | 3,47 | 6 |
| 25,88 | 3,44 | 10 |
| 26,36 | 3,38 | 100 |
| 26,67 | 3,34 | 21 |
| 26,85 | 3,32 | 15 |
| 27,96 | 3,19 | 7 |
| 28,24 | 3,16 | 7 |
| 28,52 | 3,13 | 5 |
| 28,79 | 3,10 | 9 |
| 29,81 | 2,99 | 5 |
| 30,11 | 2,97 | 9 |

**Tabelle 3: Röntgenpulverreflexe (bis zu 30 ° 2Θ) und Intensitäten (normalisiert) von 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester (Tetrahydrat)**

| **2 Θ [°]** | **d [Å]** | **I/Iₒ [%]** |
|---|---|---|
| 4,67 | 18,91 | 100 |
| 6,63 | 13,32 | 2 |
| 8,10 | 10,90 | 45 |
| 9,36 | 9,44 | 20 |
| 9,82 | 9,00 | 52 |
| 10,55 | 8,38 | 2 |
| 11,18 | 7,91 | 32 |
| 11,91 | 7,42 | 2 |
| 12,57 | 7,04 | 10 |
| 13,01 | 6,80 | 9 |
| 13,52 | 6,54 | 5 |
| 14,05 | 6,30 | 23 |
| 14,59 | 6,06 | 18 |
| 15,52 | 5,70 | 8 |
| 15,99 | 5,54 | 10 |
| 16,31 | 5,43 | 8 |
| 16,56 | 5,35 | 14 |
| 16,94 | 5,23 | 21 |
| 17,76 | 4,99 | 16 |
| 18,62 | 4,76 | 33 |
| 18,93 | 4,68 | 26 |
| 19,69 | 4,51 | 10 |
| 20,10 | 4,42 | 15 |
| 20,56 | 4,32 | 52 |
| 20,86 | 4,26 | 7 |
| 21,38 | 4,15 | 19 |
| 21,83 | 4,07 | 24 |
| 22,51 | 3,95 | 22 |
| 23,10 | 3,85 | 34 |
| 24,04 | 3,70 | 33 |
| 24,74 | 3,60 | 83 |
| 25,23 | 3,53 | 8 |
| 25,58 | 3,48 | 12 |
| 26,35 | 3,38 | 9 |
| 26,96 | 3,30 | 21 |
| 28,31 | 3,15 | 9 |
| 28,87 | 3,09 | 2 |
| 29,32 | 3,04 | 5 |
| 23,92 | 3,72 | 19 |
| 24,49 | 3,63 | 13 |
| 24,69 | 3,60 | 15 |
| 24,89 | 3,57 | 12 |
| 25,17 | 3,53 | 26 |
| 25,66 | 3,47 | 6 |
| 25,88 | 3,44 | 10 |
| 26,36 | 3,38 | 100 |
| 26,67 | 3,34 | 21 |
| 26,85 | 3,32 | 15 |
| 27,96 | 3,19 | 7 |
| 28,24 | 3,16 | 7 |
| 28,52 | 3,13 | 5 |
| 28,79 | 3,10 | 9 |
| 29,81 | 2,99 | 5 |
| 30,11 | 2,97 | 9 |

In den voranstehenden Tabellen 1 bis 3 steht der Wert "2 Θ [°]" für den Beugungswinkel in Grad und der Wert "d [Å]" für die bestimmten Abstände in Å zwischen den Gitterebenen.

Die Röntgenpulverdiagramme wurden im Rahmen der vorliegenden Erfindung mittels eines STOE STADI P - Diffraktometers, ausgerüstet mit einem ortsempfindlichen Detektor (OED) und einer Cu-Anode als Röntgenquelle und einem Primärmonochromator (CuK_{α1} - Strahlung, λ = 1.54056 Å, 40 kV, 40 mA) aufgenommen.

### Kurzbeschreibung der Abbildungen

Figuren 1 bis 3 zeigen die Röntgenpulverdiffraktogramme der drei kristallinen Formen von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester.

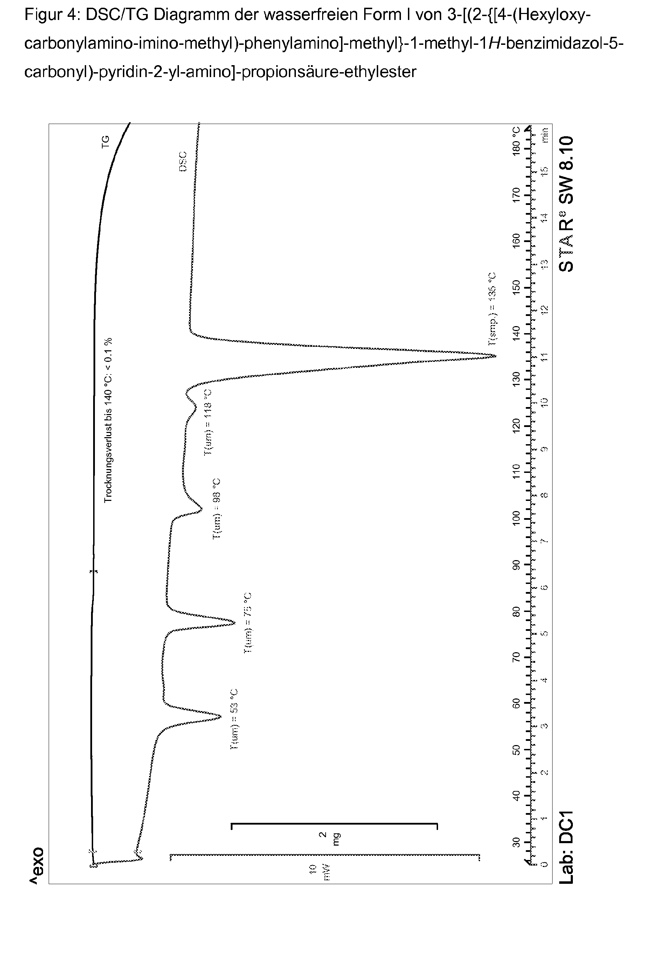

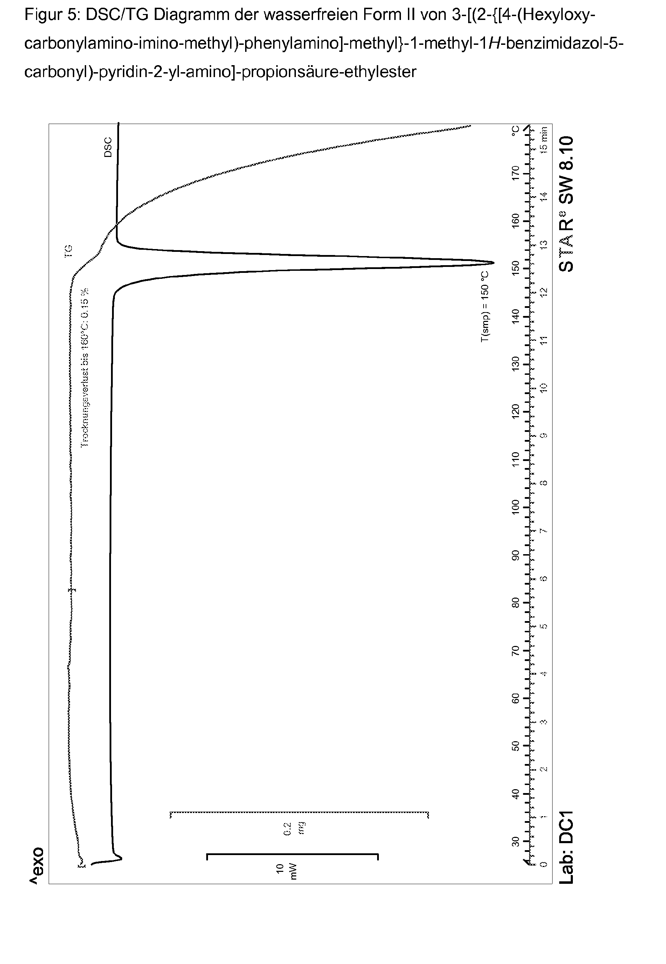

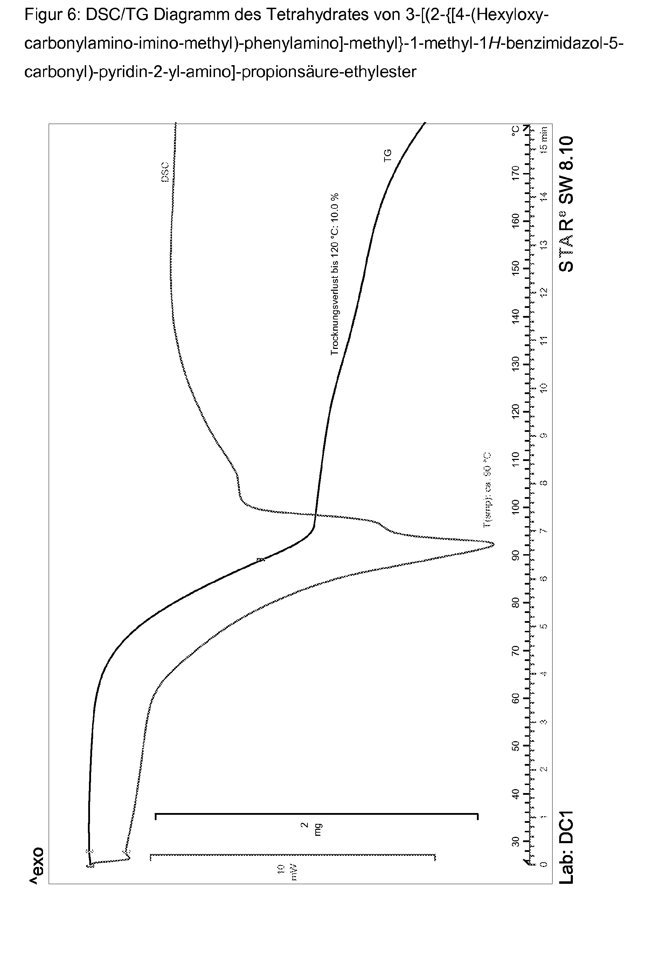

Figuren 4 - 6 zeigen die Thermoanalyse (DSC/TG) für die drei kristallinen Formen von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester.

### Beispiele

Die Schmelzpunkte wurden mittels DSC ermittelt, es wurde hierfür ein Gerät der Fa. Mettler-Toledo (Typ: DSC 821) eingesetzt. Als Schmelztemperatur wurde die Peak-Temperatur des entsprechenden Schmelzpeaks im DSC-Diagramm verwendet. Die Genauigkeit der angegebenen Schmelzpunkte beträgt etwa ± 3 °C, beim Tetrahydrat ± 5 °C, da das Tetrahydrat beim Schmelzen das in das Kristallgitter eingeschlossene Kristallwasser abgibt und dadurch ein sehr stark verbreitertes endothermes Signal resultiert.

Die Ausgangsverbindung 3-[(2-{[4-(Amino-hexyloxycarbonylimino-methyl)-phenyl-amino]-methyl}-1-methyl-1*H*-benzimidazol-5-carbonyl)-pyridin-2-yl-amino]-propionsäure-ethylester kann beispielsweise wie in der internationalen Anmeldung WO 98/37075, Beispiel 113, beschrieben hergestellt werden.

### Beispiel 1

3-[(2-{[4-(Amino-hexyloxycarbonylimino-methyl)-phenylamino]-methyl}-1-methyl-1*H-*benzimidazol-5-carbonyl)-pyridin-2-yl-aminol-propionsäure-ethylester (wasserfreie Form I) 1500 g (2,389 Mol) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethyl-ester - Base (wie in der WO 98/37075 beschrieben hergestellt) werden in 12 Liter Essigsäureethylester unter Rückfluß gelöst. Die Lösung auf 30-35 °C abkühlen. Nach wenigen Minuten begann das Produkt auszukristallisieren. Es wurde noch 60 Minuten lang bei 30-35 °C und weitere 60 Minuten bei 15-20 °C gerührt, dann der Niederschlag abgesaugt, mit 3 Liter Essigsäureethylester gewaschen und bei 40-50 °C im Umluft-Trockenschrank getrocknet.

### Ausbeute: 88,5 % der Theorie

### Beispiel 2

3-[(2-{[4-(Amino-hexyloxycarbonylimino-methyl)-phenylamino]-methyl}-1-methyl-1*H-*benzimidazol-5-carbonyl)-pyridin-2-yl-amino]-propionsäure-ethylester (wasserfreie Form II)
2.0 g 3-[(2-{[4-(Hexyloxycarbonylamino-imino-methyl)-phenylamino]-methyl}-1-methyl-1 H-benzimidazol-5-carbonyl)-pyridin-2-yl-amino]-propionsäure-ethyl-ester-Base (wie in der WO 98/37075 beschrieben hergestellt) wurden mit 10 ml Essigsäureethylester versetzt. Die hieraus resultierende Suspension wurde auf 80 °C erwärmt (Substanz geht in Lösung) und 1 Stunde unter Rückfluss gekocht. Nach ca. 30 Minuten unter Rückfluß fängt Form II an zu kristallisieren. Anschließend wurde die angefallene Substanz abfiltriert und an der Luft getrocknet.

### Ausbeute: 85 % der Theorie

### Beispiel 3

3-[(2-{[4-(Amino-hexyloxycarbonylimino-methyl)-phenylamino]-methyl}-1-methyl-1*H-*benzimidazol-5-carbonyl)-pyridin-2-yl-amino]-propionsäure-ethylester - Tetrahydrat 0.5 g 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethyl-ester-Base (wie in der WO 98/37075 beschrieben hergestellt) werden in 5 ml einer Mischung aus Aceton/Wasser = 80 : 20 bei 60 °C unter Schütteln gelöst. Lösung wurde auf ca. 30 °C abgekühlt und über einen Filter, beispielsweise über ein Sartorius Minisart-Filter SRP 15 in ein 10 ml Glasvial, filtriert und die Flasche verschlossen. Die Lösung wurde anschließend in einem Eis-/Ethanolgemisch auf ca. - 9 °C abgekühlt. Substanz begann von alleine zu kristallisieren. Nach ca. 30 Minuten im Eisbad wurden ca. 3 ml auf -9 °C abgekühlte Mischung Aceton /Wasser (80 : 20) zugesetzt, die Mischung aufgeschüttelt und anschließend über einen Filter, beispielsweise ein Rundfilter Schleicher & Schüll Nr. 595, abgesaugt.

Es wurde mit weiteren ca. 5 ml auf -9 °C abgekühlter Mischung Aceton/Wasser (80 : 20) nachgespült. Die abfiltrierte Substanz wurde vom Rundfilter in eine Kristallisierschale abgeschabt und an der Luft bei Raumtemperatur getrocknet.

### Ausbeute: 97 % der Theorie

### Beispiel 4

**Trockenampulle mit 75 mg Wirkstoff pro 10 ml**

| Zusammensetzung: | |
|---|---|
| Wirkstoff | 75,0 mg |
| Mannitol | 50,0 mg |
| Wasser für Injektionszwecke | ad 10,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 5

**Trockenampulle mit 35 mg Wirkstoff pro 2 ml**

| Zusammensetzung: | |
|---|---|
| Wirkstoff | 35,0 mg |
| Mannitol | 100,0 mg |
| Wasser für Injektionszwecke | ad 2,0 ml |

Herstellung:
Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

### Beispiel 6

Tablette mit 50 mg Wirkstoff

| Zusammensetzung: | |
|---|---|
| (1) Wirkstoff | 50,0 mg |
| (2) Milchzucker | 98,0 mg |
| (3) Maisstgrke | 50,0 mg |
| (4) Polyvinylpyrrolidon | 15,0 mg |
| (5) Magnesiumstearat | 2,0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreBt, biplan mit beidseitiger Facette und einseitiger Teilkerbe. Durchmesser der Tabletten: 9 mm.

### Beispiel 7

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 8

Kapseln mit 50 mg Wirkstoff

| Zusammensetzung: | |
|---|---|
| (1) Wirkstoff | 50,0 mg |
| (2) Maisstgrke getrocknet | 58,0 mg |
| (3) Milchzucker pulverisiert | 50,0 mg |
| (4) Magnesiumstearat | 2,0 mg |
| | 160,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel 9

Kapseln mit 350 mg Wirkstoff

| Zusammensetzung: | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mq |
| | 430,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Gr6Be 0 abgefüllt.

### Beispiel 10

Suppositorien mit 100 mg Wirkstoff

| 1 Zäpfchen enthält: | |
|---|---|
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mq |
| | 2 000,0 mg |

### Beispiel 11

| | Prozentuale Zusammensetzung | | | | pro Kapsel [mg] | pro Kapsel [mg] |
|---|---|---|---|---|---|---|
| | Kern-material | Isolier-schicht | Wirkstoff schicht | Gesamt | | |
| Weinsäure | 61,3 | - | - | 61,3 | 176,7 | 353,4 |
| Gummi arabicum | 3,1 | 2,8 | | 5,9 | 17,0 | 34,0 |
| Talkum | - | 5,6 | 3,2 | 8,8 | 25,4 | 50,7 |
| Hydroxypropylcellulose | - | - | 4,0 | 4,0 | 11,5 | 23,1 |
| Wirkstoff (bezogen auf die Base) | - | - | 20,0 | 20,0 | 50,0 | 100,0 |
| Summe | | | | 100,0 | 288,3 | 576,5 |

### Beispiel 12

| | Prozentuale Zusammensetzung | | | | pro Kapsel [mg] | pro Kapsel [mg] |
|---|---|---|---|---|---|---|
| | Kern-material | Isolierschicht | Wirkstoffschicht | Gesamt | | |
| Weinsäure | 38,5 | - | - | 38,5 | 55,5 | 166,5 |
| Gummi arabicum | 1,9 | 1,7 | | 3,6 | 5,2 | 15,6 |
| Talkum | - | 3,5 | 6,4 | 9,9 | 14,3 | 42,8 |
| Hydroxypropylcellulose | - | - | 8,0 | 8,0 | 11,5 | 34,6 |
| Wirkstoff (bezogen auf die Base) | - | - | 40,0 | 40,0 | 50,0 | 150,0 |
| Summe | | | | 100,0 | 144,2 | 432,5 |

Die Herstellung und der Aufbau der Pellets gemäß Beispiel 11 und 12 wird in der WO 03/074056 ausführlich beschrieben.

## Patentansprüche

1. 3-[(2-{[4-(Hexyloxycarbonylamino-imino-methyl)-phenylamino]-methyl}-1-methyl-1 H-benzimidazol-5-carbonyl)-pyridin-2-yl-amino]-propionsäure-ethylester - Tetrahydrat in kristalliner Form, **gekennzeichnet durch** einen Schmelzpunkt von T_{Smp}. = 90 ± 5°C unter gleichzeitiger Abgabe des ins Kristallgitter eingeschlossenen Kristallwassers (Tetrahydrat) (bestimmt über DSC; Auswertung über Peak-Maximum; Heizrate: 10°C/min).

2. Verwendung einer Verbindung gemäß Anspruch 1 zur Herstellung eines Arzneimittels mit einer die Thrombinzeit verlängernder Wirkung.

3. Verwendung einer Verbindung gemäß Anspruch 1 zur Herstellung eines Arzneimittels zur Prophylaxe von Venenthrombosen und Schlaganfall.

4. Pharmazeutische Zusammensetzung enthaltend eine Verbindung gemäß Anspruch 1 neben gegebenenfalls einem oder mehreren inerten Trägerstoffen und/oder Verdünnungsmitteln.

5. Verfahren zur Herstellung eines Arzneimittels gemäß Anspruch 4, **dadurch gekennzeichnet, dass** auf nichtchemischem Wege eine Verbindung gemäß Anspruch 1 in einen oder mehrere inerte Trägerstoffe und/oder Verdünnungsmittel eingearbeitet wird.

6. Verfahren zur Herstellung des Tetrahydrats von 3-[(2-{[4-(Hexyloxycarbonyl-amino-imino-methyl)-phenylamino]-methyl}-1-methyl-1*H*-benzimidazol-5-carbonyl)-pyridin-2-yl-amino]-propionsäure-ethylester gemäß Anspruch 1, **dadurch gekennzeichnet, daß** man
a) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base unter Schütteln bei einer Temperatur von ca. 60 °C in Aceton/Wasser (80 : 20) löst,
b) die Lösung auf eine Temperatur von ca. 30 °C abkühlt und in ein verschließbares Gefäß filtriert,
c) das verschlossene Gefäß mit der Lösung auf eine Temperatur von ca. - 9 °C abkühlt und ca. 30 Minuten lang bei dieser Temperatur beläßt,
d) ein auf -9 °C vorgekühltes Gemisch aus Aceton und Wasser (80 : 20) zugibt und die Mischung aufschüttelt,
e) die ausgefallenen Kristalle absaugt und mit einem auf -9 °C gekühlten Gemisch aus Aceton und Wasser (80 : 20) wäscht und
f) das so erhaltene Produkt an der Luft bei Raumtemperatur trocknet.

7. 3-[(2-{[4-(Hexyloxycarbonylamino-imino-methyl)-phenylamino]-methyl}-1-methyl-1 H-benzimidazol-5-carbonyl)-pyridin-2-yl-amino]-propionsäure-ethylester - Tetrahydrat, erhältlich nach dem Verfahren gemäß Anspruch 6.
